# EUROPEAN PATENT SPECIFICATION

(11) **EP 0 326 517 B1**
(45) Date of publication and mention of the grant of the patent: **06.07.1994**
(21) Application number: 89810046.6
(22) Date of filing: 19.01.1989
(51) Int. Cl.: C08B 30/12, A61K 9/48

(54) **Method of producing destructurized starch**
Verfahren zur Herstellung von destrukturierter Stärke
Procédé de préparation d'amidon destructuré

(30) Priority: 25.01.1988 GB 8801562
(43) Date of publication of application: 02.08.1989
(73) Proprietor: WARNER-LAMBERT COMPANY, Morris Plains New Jersey 07950 (US)
(72) Inventor: Stepto, Robert Frederick Thomas, Poynton Cheshire SK12 1JJ (GB); Dobler, Beat, CH-4054 Basel (CH)
(74) Representative: Silbiger, Jakob, Dr.

(56) References cited:
- EP-A- 0 118 240
- EP-A- 0 282 451
- US-A- 2 417 969

## Description

The present invention refers to a method of producing destructurized starch.

It is known that natural starch which is found in vegetable products and which contains a defined amount of water, can be treated at an elevated temperature and in a closed vessel, thereby at elevated pressure, to form a melt. The process is conveniently carried out in an injection moulding machine or extruder. The starch is fed through the hopper onto a rotating, and, for injection moulding, a rotating and reciprocating screw. The feed material moves along the screw towards the tip. During this process, its temperature is increased by means of external heaters around the outside of the barrel and by the shearing action of the screw. Starting in the feed zone and continuing in the compression zone, the particulate feed becomes gradually molten. It is then conveyed through the metering zone, where homogenization of the melt occurs, to the end of the screw. The molten material at the tip can then be further treated by injection moulding or extrusion or any other known technique to treat thermoplastic melts, to obtain shaped articles.

This treatment, which is described in the European Patent Application No. 84 300 940.8 (Publication No. 118 240) yields an essentially destructurized starch. The reason for this being that the starch is heated above the melting and glass transition temperatures of its components so that they undergo endothermic transitions. As a consequence a melting and disordering of the molecular structure of the starch granules takes place, so that an essentially destructurized starch is obtained. The expression "destructurized starch" defines starch obtained by such thermoplastic melt formation.

EP-A-0 282 451 describes the preparation of a starch melt from a composition comprising a chemically non-modified starch material, water and a chain scission catalyst whereby the mass average molar mass of the starch is reduced by a factor of 2 to 5000.

US-A-2 417,969 describes a method of treating starch comprising forming a slurry of raw starch having a concentration of about 5 to about 30% solids and repeatedly pumping the slurry under pressure through a restricted orifice to heat the slurry to a gelatinising temperature as the starch is subjected to attrition in passing through the orifice.

Although articles obtained by injection moulding of natural starch are useful, it has been found, that the shaped articles obtained therefrom can show a relatively low physical strength. It has further been found that the process itself shows a relatively high instability due to the high dependency of the melt viscosity on the shear rate within the screw barrel which renders the processing for example by injection moulding or extrusion more sensitive to conditions of screw speed, temperature, pressure and/or water content and reduces the average quality of the obtained article.

In this mentioned process of injection moulding starch, there are two important steps, namely (A) the destructurizing step, i.e. to heat the starch granules above the melting points and the glass transition temperatures of their components to effect the high temperature transitions of the molecular structure to form the melt and (B) the fabricating step, i.e. to form the melt into shaped article e.g. by injection moulding or extrusion through a die.

The properties of the shaped articles produced largely depend on the quality of the melt obtained in the destructurizing step just before said melt is formed into such shaped articles.

EP-A-0 118 240 as mentioned above discloses destructurized starch and a method for its production. US-A-2 417 969 discloses a method of treating starch in a slurry in order to produce gelatinized starch. EP-A-0 282 451 discloses a method of making destructurized starch by the addition of a chain scission catalyst. EP-A-0 298 920, published on January 11, 1989, and claiming a priority of July 7, 1987, refers to a process of removing free electrolytes or electrolytes which are associated with the phosphate groups which may be present in certain starches, in order to reduce the melting temperature of starch. None of these documents mentions the use of an endothermic transition as determined by DSC-analysis to control the destructurization process of starch.

It has now been surprisingly found, that starch with a water content within the range of 5 to 40% by weight based on the weight of the composition undergoes a "specific narrow endothermic transition" on heating to elevated temperatures and in a closed volume just prior to its endothermic changes characteristic of oxidative and thermal degradation. This specific endothermic transition can be determined by differential scanning calorimetric analysis (DSC), and is indicative on the DSC diagram by a specific relatively narrow peak just prior to the endotherm change characteristic of oxidative and thermal degradation. This peak disappears as soon as the mentioned specific endothermic transition has been undergone.

It has further been found that this last endothermic transition prior to thermal and oxidative degradation plays an important role in melt formation. A starch/water melt formed before this specific endothermic transition is undergone is opaque. If however the melt formed initially is heated further until the peak in DSC is indicating that the described "specific endothermic transition" has disappeared then the melt becomes transparent.

There are broad endotherms existing from room temperature upwards known in the prior art from DSC analysis and they have been given interpretations indicating transitions in connection with theoretical considerations relating to the melting points and glass transition temperatures of the components of starch. The newly discovered endothermic upper-transition is of a similar type but it has not been possible so far to give it a definite interpretation and this requires further investigation.

According to this present invention, it is the mentioned transparent melt which produces articles of improved physical strength with a much decreased number of defects and considerably improves the processability of starch/water compositions as described herein.

The present invention refers to a process for forming a molten composition for the use in the manufacture of shaped articles, comprising the steps of:
a) providing a solid starch composition comprising a starch composed mainly of amylose and/or amylopectin and water wherein said water content is within the range of 5% to 40% by weight based on the weight of said composition, said starch when examined by differential scanning calorimetric analysis (DSC) having a thermogram which exhibits a final narrow endothermic transition just prior to the endothermic change characteristic of oxidative and thermal degradation of the starch;
b) determining by DSC the temperature of said final narrow endothermic transition;
c) heating said composition in a screw and barrel of an injection molding machine or an extruder to a temperature within the range of 80°C to 200°C to form a melt thereof whereby the pressure created in the barrel corresponds at a minimum to the composition is from 5 to 40% by weight with respect to the weight of the composition,
   characterised in that the composition is heated until all endothermic transitions, as determined by differential scanning calorimetry, prior to that endothermic transition which is characteristic of the said starch's oxidative and thermal decomposition have been traversed.
   The present invention also refers to the melt obtained by said process as well as to any solid shapes or particles obtained from said melt. It further refers to the discovery of factors which reduce the vapor pressure of water at the used temperature;
   characterized in that
d) the set temperature and residence time of heating in step c) is selected so that the composition is heated to the temperature determined in step b) until all endothermic transitions up to and including said final narrow endothermic transition of the starch have been traversed.

The present invention also refers to the melt obtained by said process as well as to any solid shapes or particles obtained from said melt. It further refers to the discovery of factors which reduce the temperature and the enthalpy of the transition and hence aid processing.

The melt obtained by the process as described herein is a thermoplastic melt and can be treated accordingly.

The term "starch" as used herein includes chemically non-modified starch as for example generally carbohydrates of natural, vegetable origin, composed mainly of amylose and /or amylopectin. They may be extracted from various plants, examples being potatoes, rice, tapioca, corn, and cereals such as rye, oats and wheat. It further include physically modified starch such as gelatinized or cooked starch.

Such starch is suitably heated for destructurization in a screw/barrel of an extruder for a time long enough to effect destructurization. The set temperature is preferably within the range of 120°C to 190°C, preferably within the range of 120°C to 190°C, preferably within the range of 130°C to 190°C depending on the type of starch used. For this destructurization, the starch material is heated preferably in a closed volume. A closed volume can be a closed vessel or the volume created by the sealing action of the unmolten feed material as happens in the screw of injection moulding or extrusion equipment. In this sense the screw and barrel of an injection moulding machine or an extruder are to be understood as being a closed vessel. Pressures created in a closed vessel correspond to the vapour pressure of water at the used temperature but of course pressure may be applied and/or generated as normally occurs in a screw and barrel. The preferred applied and/or generated pressures are in the range of the pressures which occur in extrusion or injection moulding processes and are known per se, i.e. from zero to 150 x 10⁵ N/m² preferably from zero to 75 x 10⁵ N/m² and most particularly from zero to 50 x 10⁵ N/m².

As is known to those skilled in the art, the melt formation behaviour, i.e. the rate of melt formation, viscosities etc. in a screw and barrel depend on many factors, such as the size of the barrel, its length and diameter, screw design, speed of rotation, heating profile, etc.

It is therefore necessary to determine for every machine used the set temperatures and residence times necessary to achieve melt formation according to this invention, i.e. a melt that does not show anymore the specific final narrow endothermic transition in its DSC thermogram.

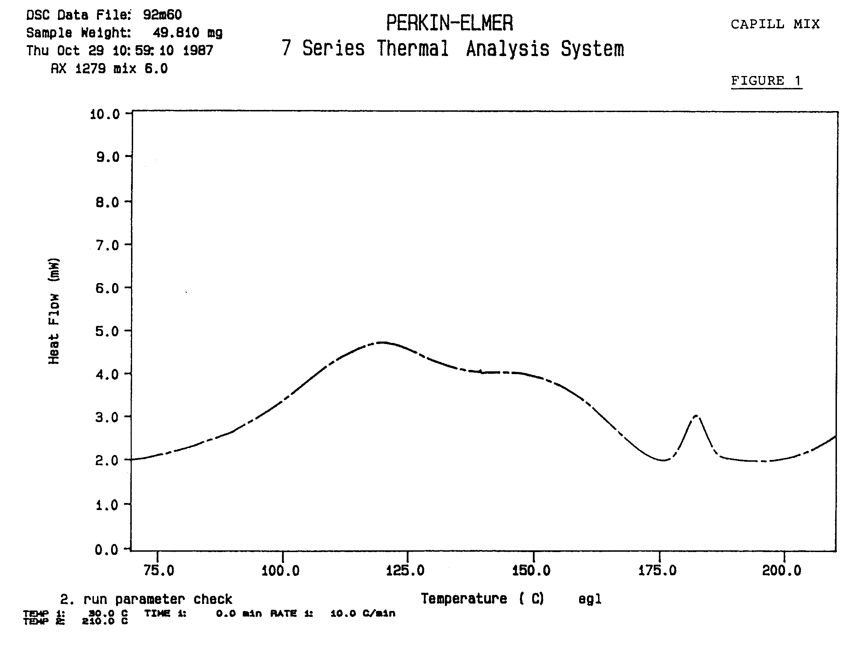

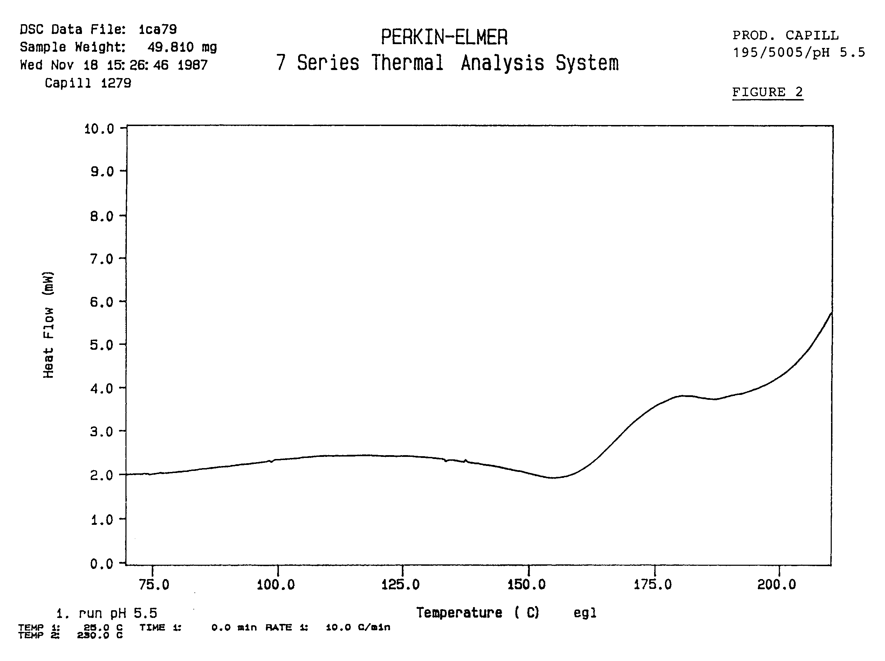

The disappearance of the transition during processing can simply be ascertained by obtaining the DSC thermogram of the moulded material and comparing it with that of the unmoulded material obtained at the same scan rate. Such a comparison is made in Figure 1 and 2. Figure 1 shows a DSC thermogram of a moulding mixture consisting principally of native starch and water run at 10°C per minute. The upper-transition is marked A. Figure 2 shows the thermogram of the same mixture after injection moulding. The sharp upper-transition is absent and, from around 160°C upwards, is replaced by a general increase in the heat flow and a broader melt-formation characteristic of once-processed material. The broader transition probably results from the new morphology of the material, once it is cooled after injection moulding.

If the upper transition has not disappeared after processing then, in order to obtain a truly homogeneous melt, processing conditions must be changed, i.e. set temperatures, residence time and speed of screw rotation increased, until its disappearance on processing is achieved.

The reasons for this procedure are two-fold:
1. It is well-known that the nominal temperatures registered by DSC equipment are, because of the finite heat capacities of the sample and holders and the finite rates of heating used, not the temperatures of the samples.
2. It is well-known that the temperatures of material in the screw of an extruder or injection moulding machine are, because of heats of melt-formation, structural changes and the shearing action of the screw, not the same as the set temperatures of the barrel.

Therefore, the nominal temperatures and residence times during DSC measurements and set temperatures and residence times during processing are not equivalent. For example, the material in Figure 2 had been processed by injection moulding, increasing from room temperature to a set temperature of 155°C over 500 seconds. However, the thermogram in Figure 1 shows an upper transition at a nominal temperature of 182°C, the temperature having been increased from 30°C to 180°C in 900 seconds. In spite of the lower set temperature and shorter time of the processing compared with that of the DSC measurements, the upper transition has been undergone due to the real temperature in the DSC sample being lower than the nominal temperature and the real temperature of the material during processing being higher for the aforesaid reasons.

The essentially destructurized starch/water composition according to the present invention has a preferred water content in the range of 10 to 20% by weight of the composition, preferably 12% to 19% and especially 14% to 18% by weight, calculated to the weight of the composition.

The starch/water composition according to this invention is heated to a temperature which is generally within the range of 80 to 200°C, preferably within the range of 120 to 190°C and especially at 130 to 190°C.

The minimum pressure for forming the melt corresponds to the water vapour pressure produced at these temperatures. The process is carried out in a closed volume as explained above, and under the range of pressures which are used in extrusion or injection molding processes and known per se, e.g. from zero to 150 x 10⁵ N/m² preferably from zero to 75 x 10⁵ N/m² and most particularly from zero to 50 x 10⁵ N/m².

When forming a shaped article by extrusion the pressures are preferably as mentioned above. If the melt of the destructurized starch composition according to this invention is e.g. injection molded, the normal range of injection pressures used in injection molding is applied, namely from 300 x 10⁵ N/m² to 3.000 x 10⁵ N/m² preferably 700 x 10⁵ - 2200 10⁵ N/m².

The starch material of the present invention may contain or may be mixed with additives such as extenders, lubricants, plasticizers and/or coloring agents.

Additives having different hydrophobic/hydrophilic characteristics may be used to reduce the enthalpy of the upper-transition and, hence, to aid processing. The additional endotherm at lower temperatures generally is the melting of the additives and is at too low a temperature to affect melt homogeneity.

These additives may be added before the destructurizing step or after this step i.e. mixed with the solid granules of the destructurized starch. It mainly depends on the intended use of the destructurized starch.

Such additives are extenders of different kinds, e.g. gelatin, vegetable proteins such as sunflower protein, soybean proteins, cotton seed proteins, peanut proteins, rape seed proteins, blood proteins, egg proteins, acrylated proteins; water-soluble polysaccharides such as:
alkylcelluloses hydroxyalkylcelluloses and hydroxyalkylalkylcelluloses, such as: methylcellulose, hydroxymethylcellulose, hydroxyethylcellulose, hydroxypropylcellulose, hydroxyethylmethylcellulose, hydroxpropylmethylcellulose, hydroxybutylmethylcellulose, cellulose esters and hydroxyalkylcelluloseesters such as:
cellulose acetylphtalate (CAP), Hydroxypropylmethylcellulose (HPMCP); carboxyalkylcelluloses, carboxyalkylalkylcelluloses, carboxyalkylcellulose esters such as : carboxymethylcellulose and their alkalimetal salts; water-soluble synthetic polymers such as: polyacrylic acids and polyacrylic acid esters, polymethyacrylic acids and polymethacrylic acid esters, polyvinylacetates, polyvinylalcohols, polyvinylacetatephthalates (PVAP), polyvinylpyrrolidone, polycrotonic acids; suitable are also phtalated gelatin, gelatin succinate, crosslinked gelatin, shellac, water soluble chemical derivatives of starch, cationically modified polymers of acrylates and methacrylates possessing, for example, a tertiary or quaternary amino group, such as the diethylaminoethyl group, which may be quaternized if desired; and other similar polymers.

Such extenders may optionally be added in any desired amount preferably up to and including 50 %, preferably within the range of 3 % to 10 % based on the weight of all components.

Further additives are inorganic fillers, such as the oxides of magnesium, aluminum, silicon, titanium, etc. preferably in a concentration in the range of 0.02 to 3 % by weight preferably 0.02 to 1 % based on the weight of all components.

Further examples of additives are plasticizers which include polyalkylene oxides, such as polyethylene glycols, polypropylene glycols, polyethylene-propylene glycols; organic plasticizers with low molecular weights, such as glycerol, glycerol monoacetate, diacetate or triacetate; propylene glycol, sorbitol, sodium diethylsulfosuccinate, triethyl citrate, tributyl citrate, etc., added in concentrations ranging from 0.5 to 15 %, preferably ranging from 0.5 to 5 % based on the weight of all the components.

Examples of coloring agents include known azo dyes, organic or inorganic pigments, or coloring agents of natural origin. Inorganic pigments are preferred, such as the oxides of iron or titanium, these oxides, known per se, being added in concentrations ranging from 0.001 to 10 %, preferably 0.5 to 3 %, based on the weight of all the components.

The sum of the plasticizer and water contents should preferably not exceed 25 %, and should most preferably not exceed 20 %, based on the weight of all the components.

There may further be added compounds to improve the flow properties of the starch material such as animal or vegetable fats, preferably in their hydrogenated form. These fats have preferably a melting point of 50°C or higher. Preferred are Triglycerides with C₁₂ -, C₁₄ -, C₁₆ -, and C₁₈ - fatty acids.

These fats can be added alone without adding extenders or plasticizers.

These fats can advantageously be added alone or together with mono- and/or diglycerides or phosphatides, especially lecithin. The mono- and diglycerides are preferably derived from the types of fats described above, i.e. with C₁₂ -, C₁₄ -, C₁₆ -, and C₁₈ - fatty acids.

The total amounts used of the fats mono-, diglycerides and/or lecithins are up to 5 % and preferably within the range of about 0.5 to 2 % by weight of the total composition.

It is further recommended to add silicon dioxide or titanium dioxide in a concentration of 0.02 to 1 % by weight of the total composition. These compounds act as texturizing agent.

The materials described herein above form on heating and in a closed vessel a melt with thermoplastic properties, i.e. under controlled water-content and pressure conditions. Such a melt can be used in various techniques just like thermoplastic materials. These techniques include injection molding, blow molding, extrusion and coextrusion (rod, pipe and film extrusion), compression molding, to produce known articles as produced with these techniques. These articles include bottles, sheets, films, packaging materials, pipes, rods, laminates, sacks, bags, pharmaceutical capsules.

The following examples further explain the invention.

### Example 1

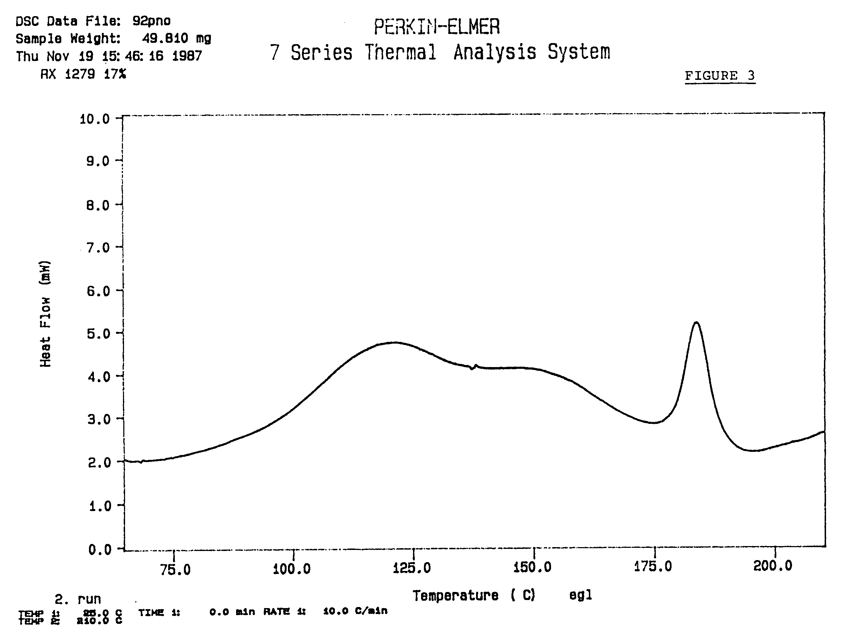

A sample of native potato starch containing 17% water on a total weight basis was heated in a closed pan of a DSC at a rate of 10°C per minute. The obtained thermogram is shown in Fig. 3. The upper-transition occurs at a nominal temperature of 183°C. Two samples of the same material were heated separately under the said conditions. The first sample was heated to a nominal temperature of 195°C just after the transition is completed. After cooling and opening the pan of the DSC, the sample was found as a solidified transparent melt. A second sample was heated under the same conditions to a nominal temperature of only 172°C. After cooling and opening the pan of the DSC an opaque material was found, showing incomplete melt formation.

### Example 2

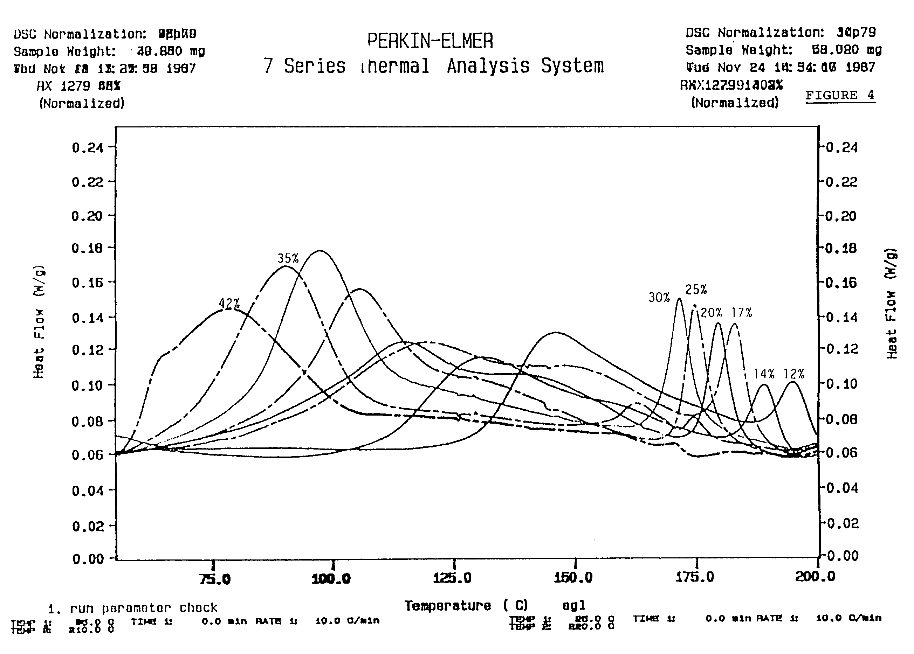

DSC-thermograms were prepared from native starches with 12%, 14%, 17%, 20%, 25%, 30%, 35% and 42% water content (weight % based on a total weight basis). The result are shown in Figure 4. It is clear that the upper-transition occurs up to a water content of about 40%.

### Example 3

Different samples of potato starch were used to produce small, thin-walled (ca. 0.3 mm) pharmaceutical containers by injection moulding with the set temperatures of the barrel increasing from room temperature to 160°C over 9 1/2 minutes. The samples were chosen because they had a range of nominal upper-transition temperatures of 14°C (183°C - 197°C) as measured by DSC at 10°C per minute. The moulded containers were inspected. The % of defective mouldings are listed in table 1 in relation to their upper-transition temperatures. The % defective parts decrease with decrease in upper-transition temperature of the material.

It should be remembered that the true temperature of the melt during processing is higher than the set temperature and that the upper-transition endotherm has a range of about 20°C and a nominal temperature higher than the true temperature. In the light of this and from the results in table 1, it can be seen that the lower is the nominal upper-transition temperature of the material, the higher is the percentage of its melt which has undergone the upper endothermic transition during processing and the lower is the resulting % defective parts.

**Table 1**

| Sample No. | nominal upper transition temp. °C | set temperature | % defective parts |
|---|---|---|---|
| 1 | 197 | 160°C | 6.8 |
| 2 | 190 | " | 6.8 |
| 3 | 187,5 | " | 4.4 |
| 4 | 187 | " | 3.6 |
| 5 | 185,5 | " | 2.6 |
| 6 | 185 | " | 2.3 |
| 7 | 183,5 | " | 1.0 |
| 8 | 183 | " | 0.6 |

### Example 4

The upper-transition temperature may be controlled by the concentration and type of counterions in the starch. Table 2 and table 3 show the transition temperatures of two starches, in the native state and with the metallic cations in that state removed or replaced by other cations by washing at room temperature, so that no thermal destructuring or degradation occurs.

**Table 2**

| Sample No. | treatment | DSC, 10°C/min., nominal upper-transition temp. |
|---|---|---|
| 9 | none | 197°C |
| 10 | washed with water to eliminate free salts | 185°C |

Sample 9 is a native starch found to contain 1 g of free salts per 5 kg of starch. This starch was washed at room temperature with an excess of de-mineralized water to remove said salt as determined by gravimetric analysis to obtain Sample No. 10, which shows a reduction of 12°C of the upper-transition temperature compared with the corresponding native material of Sample No. 9. The processing of the starch of Sample 9 at lower temperature is thus facilitated by washing with water.

A second native starch (Sample No. 11) was washed with excess 0.1 M HCl at room temperature and then neutralized with the appropriate amount of 0.1 M base to give the cationic starches Samples Nos 12 to 15 in which the native cations associated with the phosphate groups of the starch are replaced by the single cationic species as indicated in table (Samples Nos 12 to 15). In this case the upper-transition temperature is surprisingly increased depending upon the species of cation.

**Table 3**

| Sample No. | cation added after washing | DSC, 10°C/min., nominal upper-transition temp. |
|---|---|---|
| 11 | none | 183°C |
| 12 | NH₄⁺ | 199°C |
| 13 | Ca⁺² | 200°C |
| 14 | Na⁺ | 211°C |
| 15 | K⁺ | 212°C |

For Sample 10 processing is facilitated compared with the native material. For Samples 12 to 15 processing is more difficult compared to that of the native material.

### Example 5

A native starch (17% water content) was mixed with 1% of a mixture of mono-, di- and triglycerides of fatty acids having a distribution of chain lengths and 0,5% solid soya lecithin. The amounts of additives were calculated on the basis of the native starch alone.

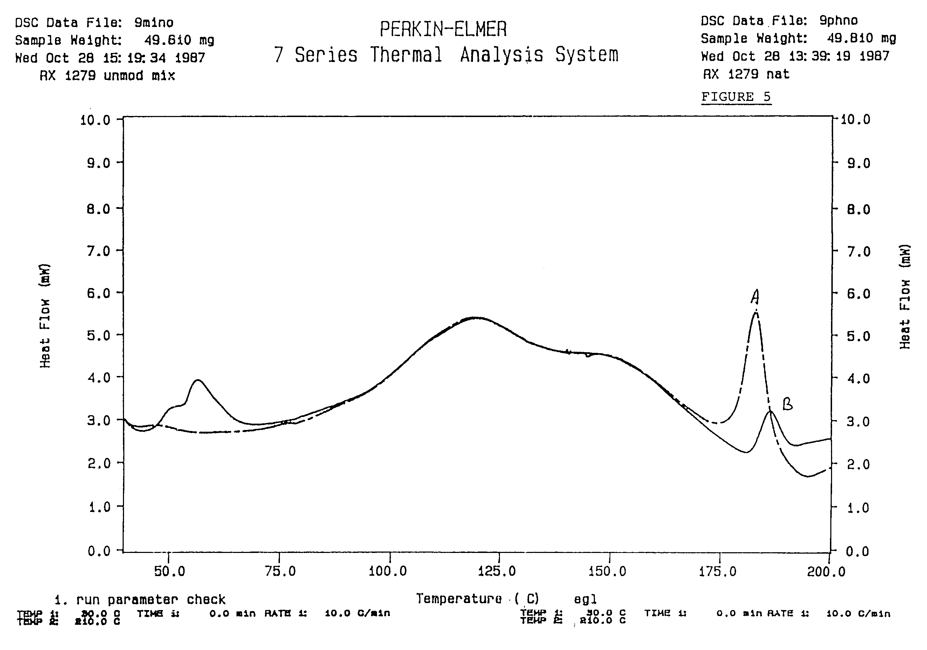

Figure 5 shows the DSC thermograms of this native mixture (curve B) and of potato starch plus 17% water (curve A).

The enthalpy of the upper-transition is much less for curve B, hence, processing is facilitated. The additional endotherm at lower temperatures for curve B is the melting of the additives and is at too low a temperature to affect melt homogeneity.

## Claims

1. A process for forming a molten composition for the use in the manufacture of shaped articles, comprising the steps of:
a) providing a solid starch composition comprising a starch composed mainly of amylose and/or amylopectin and water wherein said water content is within the range of 5% to 40% by weight based on the weight of said composition, said starch when examined by differential scanning calorimetric analysis (DSC) having a thermogram which exhibits a final narrow endothermic transition just prior to the endothermic change characteristic of oxidative and thermal degradation of the starch;
b) determining by DSC the temperature of said final narrow endothermic transition;
c) heating said composition in a screw and barrel of an injection molding machine or an extruder to a temperature within the range of 80°C to 200°C to form a melt thereof whereby the pressure created in the barrel corresponds at a minimum to the vapor pressure of water at the used temperature;
characterized in that
d) the set temperature and residence time of heating in step c) is selected so that the composition is heated to the temperature determined in step b) until all endothermic transitions up to and including said final narrow endothermic transition of the starch have been traversed.

2. A process according to claim 1 wherein the starch was extracted from potatos, rice, tapioca, corn, rye, oats and/or wheat.

3. A process according to claim 2 wherein the starch was extracted from potatos, rice and/or wheat.

4. A process according to claim 3 wherein the starch was extracted from potatos.

5. A process according to any one of the claims 1 to 4 wherein the starch composition is heated to a set temperature within the range of 120°C and 190°C.

6. A process according to any one of the claims 1 to 5 wherein the pressures applied are from zero to 150 x 10² N/m².

7. A process according to any one of the claims 1 to 6 wherein the starch/water composition has a water content in the range of 10% to 20% by weight of the composition.

8. A process according to any preceding claim, wherein the water content of the starch/water composition is in the range of 12 to 19% by weight of the composition.

9. A process according to any preceding claim, wherein the water content of the composition in the range of 14 to 18% by weight of the composition.

10. A process according to any preceding claim, wherein the composition is mixed with extenders, lubricants, plasticizers, inorganic fillers and/or colouring agents.

11. A process according to any preceding claim, wherein the composition is mixed with extenders in an amount up to 50%, based on the weight of all components.

12. A process according to any preceding claim, wherein the composition is mixed with plasticizers added in concentrations ranging from 0.5 to 5% based on the weight of all the components.

13. A process according to any preceding claim, wherein the composition is mixed with a compound selected from the group consisting of: polyethylene glycols, polypropylene glycols, polyethylene-propylene glycols, added in concentrations ranging from 0.5 to 5% based on the weight of all the components.

14. A process according to any preceding claim, wherein the composition is mixed with inorganic fillers in a concentration in the range of 0.02 to 3% by weight, based on the weight of all components.

15. A melt of a composition as obtained by any one of the claims 1 to 14.

16. Solid articles whenever made from a melt as claimed in claim 15.

17. A shaped article according to claim 16, made by injection moulding, blow moulding, extrusion, coextrusion or compression moulding.

18. Powders, granules, bottles, sheets, films, packaging material, pipes, rods, laminates, sacks, bags, pharmaceutical capsules, according to the claims 17 or 18.

## Patentansprüche

1. Verfahren zur Herstellung einer geschmolzenenen Zusammensetzung zum Gebrauch in der Fabrikation von geformten Gegenständen, umfassend die folgenden Schritte:
a) Vorlegen einer festen Stärkezusammensetzung, beinhaltend eine Stärke, hauptsächlich bestehend aus Amylose und/oder Amylopektin, und Wasser, wobei der Wassergehalt im Bereich von 5 bis 40 Gewichts-% liegt, bezogen auf das Gewicht der Zusammensetzung, besagte Stärke ergibt bei Untersuchung mittels Differential-Scanning-Calorimetric-Analyse (DSC) ein Thermogramm, welches einen endständigen engen endothermen Übergang aufweist, unmittelbar vor dem endothermen Wechsel, welcher für oxydativen und thermalen Abbau der Stärke bezeichnend ist;
b) Bestimmen der Temperatur des besagten endständigen engen endothermen Übergangs mittels DSC;
c) Erwärmen der Zusammensetzung in einer Schnecke und einer Trommel einer Spritzgiess- oder Extrudiervorrichtung auf eine Temperatur im Bereich von 80°C bis 200°C, um daraus eine Schmelze zu bilden, wobei der in der Trommel erzeugte Druck mindestens dem Wasserdampfdruck bei den verwendeten Temperaturen entspricht;
dadurch gekennzeichnet, dass
d) die eingestellte Temperatur und die Verweilzeit während des Erwärmens in Stufe c) so gewählt sind, dass die Zusammensetzung auf die in Stufe b) bestimmte Temperatur solange erwärmt wird, bis alle endothermen Übergänge bis und mit dem besagten endständigen engen endothermen Übergang der Stärke durchschritten worden sind.

2. Verfahren nach Anspruch 1, wobei die Stärke aus Kartoffeln, Reis, Tapioka, Mais, Roggen, Hafer und/oder Weizen gewonnen wurde.

3. Verfahren nach Anspruch 2, wobei die Stärke aus Kartoffeln, Reis und/oder Weizen gewonnen wurde.

4. Verfahren nach Anspruch 3, wobei die Stärke aus Kartoffeln gewonnen wurde.

5. Verfahren nach einem der Ansprüche 1 bis 4, wobei die Zusammensetzung auf eine eingestellte Temperatur im Bereich von 120°C bis 190°C erwärmt wurde.

6. Verfahren nach einem der Ansprüche 1 bis 5, wobei Drücke im Bereich von 0 bis 150 x 10⁵ N/m² ausgeübt werden.

7. Verfahren nach einem der Ansprüche 1 bis 6, wobei die Stärke/Wasser - Zusammensetzung einen Wassergehalt im Bereich von 10 bis 20%, bezogen auf das Gewicht der Zusammensetzung, aufweist.

8. Verfahren nach einem der vorhergehenden Ansprüche, wobei der Wassergehalt der Stärke/Wasser - Zusammensetzung im Bereich von 12 bis 19%, bezogen auf das Gewicht der Zusammensetzung, liegt.

9. Verfahren nach einem der vorhergehenden Ansprüche, wobei der Wassergehalt der Zusammensetzung im Bereich von 14 bis 18%, bezogen auf das Gewicht der Zusammensetzung, liegt.

10. Verfahren nach einem der vorhergehenden Ansprüche, wobei die Zusammensetzung mit Streckmittel, Gleitmittel, Plastifizierungsmittel, anorganischem Füllstoff und/oder Färbemittel gemischt wird.

11. Verfahren nach einem der vorhergehenden Ansprüche, wobei die Zusammensetzung mit Streckmittel in einer Menge von bis zu 50%, bezogen auf das Gewicht aller Bestandteile, gemischt wird.

12. Verfahren nach einem der vorhergehenden Ansprüche, wobei die Zusammensetzung mit Plastifizierungsmittel in einer Konzentration im Bereich von 0,5 bis 5%, bezogen auf das Gewicht aller Bestandteile, gemischt wird.

13. Verfahren nach einem der vorhergehenden Ansprüche, wobei die Zusammensetzung gemischt wird mit einer Verbindung, ausgewählt von der Gruppe umfassend: Polyäthylenglykole, Polypropylenglykole, Polyäthylen/ propylen-Glykole in einer Konzentration im Bereich von 0,5 bis 5%, bezogen auf das Gewicht aller Bestandteile.

14. Verfahren nach einem der vorhergehenden Ansprüche, wobei die Zusammensetzung mit anorganischen Füllstoffen in einer Konzentration im Bereich von 0,02 bis 3 Gewichts-%, bezogen auf das Gewicht aller Bestandteile, gemischt wird.

15. Eine Schmelze einer Zusammensetzung wie erhalten gemäss einem der Ansprüche 1 bis 14.

16. Feste Gegenstände wenn immer hergestellt aus einer wie in Anspruch 15 beanspruchten Schmelze.

17. Ein geformter Gegenstand gemäss Anspruch 15, hergestellt durch Spritzgiessen, Blasformen, Extrudieren, Coextrudieren oder Pressformen.

18. Puder, Granulate, Flaschen, Folien, Filme, Verpackungsmaterialien, Rohre, Stäbe, Verbundgebilde, Säcke, Beutel, pharmazeutische Kapseln, gemäss den Ansprüchen 21 oder 22.

## Revendications

1. Un procédé pour former une composition fondue pour l'utilisation dans la fabrication d'articles façonnés comprenant les étapes de:
a) fourniture d'une composition solide d'amidon comprenant de l'amidon, essentiellement formé d'amylose et/ou d'amylopectine, et de l'eau, caractérisé en ce que ladite teneur en eau est comprise entre 5 et 40% en poids, calculée par rapport au poids de ladite composition, ledit amidon, examiné par l'analyse de differential scanning calorimetric (DSC), a un thermogramme qui montre une transition endothermique finale étroite juste antérieure au change endothermique, caractéristique de la dégradation oxydative et thermale de l'amidon;
b) détermination par DSC de la température de ladite transition endothermique finale étroite;
c) chauffage de ladite composition dans un fût et une vis d'un appareil de moulage par injection ou d'extrusion à une température comprise entre 80°C et 200°C pour en former une masse fondue, caractérisé en ce que la pression créée dans la vis correspond au minimum à la pression de vapeur de l'eau à la température utilisée;
étant caractérisé en ce que
d) la température réglée et le temps de résidence pendant le chauffage dans l'étape c) sont choisis de sorte que la composition soit chauffée à la température déterminée à l'étape b) jusqu'à ce que toutes les transitions endothermiques, ladite transition endothermique finale étroite de l'amidon y incluse, ont été traversées.

2. Le procédé selon la revendication 1, caractérisé en ce que l'amidon a été extrait de pommes de terre, riz, tapioca, maïs, seigle, avoine et/ou froment.

3. Le procédé selon la revendication 2, caractérisé en ce que l'amidon a été extrait de pommes de terre, riz et/ou froment.

4. Le procédé selon la revendication 3, caractérisé en ce que l'amidon a été extrait de pommes de terre.

5. Le procédé selon l'une des revendications 1 à 4, caractérisé en ce que la composition d'amidon est chauffée à une température réglée comprise entre 120°C et 190°C.

6. Le procédé selon l'une des revendications 1 à 5, caractérisé en ce que les pressions appliquées sont comprises entre zéro et 150 x 10⁵ N/m².

7. Le procédé selon l'une des revendications 1 à 6, caractérisé en ce que la composition amidon/eau a une teneur en eau comprise entre 10 et 20%, par rapport au poids de la composition.

8. Le procédé selon l'une quelconque des revendications précédentes, caractérisé en ce que la composition amidon/eau a une teneur en eau comprise entre 12 et 19%, par rapport au poids de la composition.

9. Le procédé selon l'une quelconque des revendications précédentes, caractérisé en ce que la composition a une teneur en eau comprise entre 14 et 18%, par rapport au poids de la composition.

10. Le procédé selon l'une quelconque des revendications précédentes, caractérisé en ce que la composition est mélangée à des agents d'extension, des lubrifiants, des plastifiants, des charges minérales inorganiques et/ou des colorants.

11. Le procédé selon l'une quelconque des revendications précédentes, caractérisé en ce que la composition est mélangée à des agents d'extension, dans une quantité allant jusqu'à 50%, calculée par rapport au poids de tous les constituants.

12. Le procédé selon l'une quelconque des revendications précédentes, caractérisé en ce que la composition est mélangée à des plastifiants, étant ajoutés en des concentrations comprises entre 0.5 et 5%, par rapport au poids de tous les constituants.

13. Le procédé selon l'une quelconque des revendications précédentes, caractérisé en ce que la composition est mélangée à un composé choisi dans le groupe comprenant: polyéthylèneglycols, polypropylèneglycols, polyéthylène-propylèneglycols, étant ajouté en des concentrations comprises entre 0.5 et 5%, par rapport au poids de tous les constituants.

14. Le procédé selon l'une quelconque des revendications précédentes, caractérisé en ce que la composition est mélangée à des charges minérales inorganiques, en une concentration comprise entre 0.02 et 3% en poids, par rapport au poids de tous les constituants.

15. La masse fondue d'une composition comme obtenue selon l'une quelconque des revendications 1 à 14.

16. Des articles solides si produits à partir d'une masse fondue comme revendiqué dans la revendication 15.

17. Un article façonné selon la revendication 15, produit par le moulage par injection, le moulage par soufflage, l'extrusion, la coextrusion ou le moulage par compression.

18. Des poudres, des granules, des bouteilles, des feuilles, des films, des emballages, des tubes, des tiges, des stratifiés, des sacs, des sachets, des capsules pharmaceutiques, selon les revendications 21 ou 22.
